# EUROPEAN PATENT APPLICATION

(11) **EP 2 866 028 A1**
(43) Date of publication of application: **29.04.2015**
(21) Application number: 14190543.0
(22) Date of filing: 27.10.2014
(51) Int. Cl.: G01N 33/00

(54) **Apparatus and method for selectively determining a mode of operation**

(30) Priority: 28.10.2013 GB 201318987
(71) Applicant: Crowcon Detection Instruments Limited, Oxfordshire OX14 4SD (GB)
(72) Inventor: Rosso, Vince, Abingdon, Oxfordshire OX14 4SD (GB)
(74) Representative: Black, Diego

(57) **Abstract**

An apparatus for selectively switching a mode of operation of a detector (20), the apparatus comprising a detector (20) configured for connection to a signalling terminal (29), switching means (24) for configuring the detector (20) to operate in one of sink mode or source mode and a voltage comparator (26), wherein the apparatus is configured to selectively switch to operate in sink mode or source mode based on a comparison of a voltage from the signalling terminal (29) received at the voltage comparator (26) with a threshold voltage.

## Description

### Field of invention

The present invention relates to a method and apparatus for automatically determining a mode of operation of a detector, in particular a gas detector.

### Background to the invention

It is known to use 4 - 20 mA current signalling for the operation of detectors, such as gas detectors. Three-wire connection apparatus uses 4 - 20 mA current signalling (when power is supplied to the detector from a control panel). The method of signalling can be configured to operate in two modes: sink mode, or source mode. In sink mode the measuring device (i.e. the detector) signals a measured value by drawing current from the power/control device to which it is connected. In source mode, the measuring device signals a measured value by supplying current to the power/control device to which it is connected. Correct operation of a detector in either of sink or source modes typically requires user intervention to ensure that the correct detector mode is selected to match the power/control device setting, otherwise incorrect operation may result.

Traditionally, when commissioning detectors for operational use, it is necessary to manually configure the wiring of the gas detectors such that they are wired in the correct way to operate with any computational processing equipment (for example a control panel) with which they are associated. This is usually accomplished by variations in wiring *viz*. separate terminals for source and sink modes, or by using selection switches or selection links, which can be positioned to connect appropriate circuitry. Such commissioning can be time consuming. Furthermore, once commissioned the mode of the detector (sink or source) needs to be recorded for future reference. This may also be time consuming as records for each detector must be collected and maintained.

Incorrect configuration of a detector requires that either the detector is reconfigured, or the control panel to which it is connected is reconfigured. In industrial situations, where there may be many detectors connected to a control panel and/or where there are large numbers of detectors to deal with, it can be very time consuming to ensure correct operational configuration of the detectors. In a gas refinery, or the like, there may several hundred or thousand of detectors which may need to be configured.

Further, although once initially configured to operate in sink or source mode, it is likely that the mode of operation will not change for normal operation, industrial plant updates or upgrades may require that the detectors are reconfigured. Manually updating detectors to ensure correct operation is time consuming, economically expensive and error prone.

### Summary of the invention

According to an aspect of the invention, there is provided an apparatus for selectively switching a mode of operation of a detector, the apparatus comprising a detector configured for connection to a signalling terminal, switching means for configuring the detector to operate in one of sink mode or source mode and a voltage comparator, wherein the apparatus is configured to selectively switch to operate in sink mode or source mode based on a comparison of a voltage from the signalling terminal received at the voltage comparator with a threshold voltage.

Therefore an aspect of the present invention enables fast and effective switching of the mode of a detector to the correct mode of operation with no user intervention.

Beneficially, automatic detection of the correct mode of operation (source mode or sink mode) of a detector upon its installation results in a safe and reliable way to configure the detector to run without spending excessive amounts of resources on time, labour and cost. The requirement to manually check and wire each detector is removed. This is particularly advantageous in situations where there are multiple detectors, for example in industrial plants, where there may be hundreds of gas detectors and the time required to install and/or upgrade the detectors is significant.

Advantageously, the apparatus automatically switches to operate in source mode or sink mode, depending on the system to which it is being integrated. This removes the possibility of erroneous installation and the corrective action that would need to be taken, which would involve reconfiguration of the detector, or reconfiguration of the system to which the detector is being integrated.

Beneficially, since the apparatus automatically determines the correct mode of operation and switches to operate in that mode, there is no requirement to keep a record of the previous installation/commissioning of the apparatus. This saves further time and is more cost efficient.

According to a second aspect of the invention, there is provided a method for selectively switching a mode of operation of a detector, the method comprising receiving a voltage signal at a detector, at a voltage comparator, making a comparison of the voltage signal received at the detector with a threshold voltage and based on the comparison, switching to operate the detector in one of sink mode or source mode.

Further aspects of the invention will become apparent from the appended claim set.

### List of figures

Preferred embodiments of the present invention will now be described, by way of example only, with reference to the accompanying drawings, in which:
Figure 1 is a diagram of apparatus according to an example of the invention;
Figure 2a is schematic of source mode operation of a detector according to an example of the invention;
Figure 2b is a schematic of sink mode operation of a detector according to an example of the invention;
Figure 3 is a flowchart showing a method according to an example of the invention; and
Figure 4 is a diagram of apparatus for determining and switching operational modes according to an example of the invention.

### Specific description

For three-wire connection detectors, such as gas detectors, operating with 4 - 20 mA signalling, the operating mode may either be source mode, whereby the detector signals by supplying a current to a control/power panel, or sink mode, whereby the detector signals by drawing current from a control/power panel.

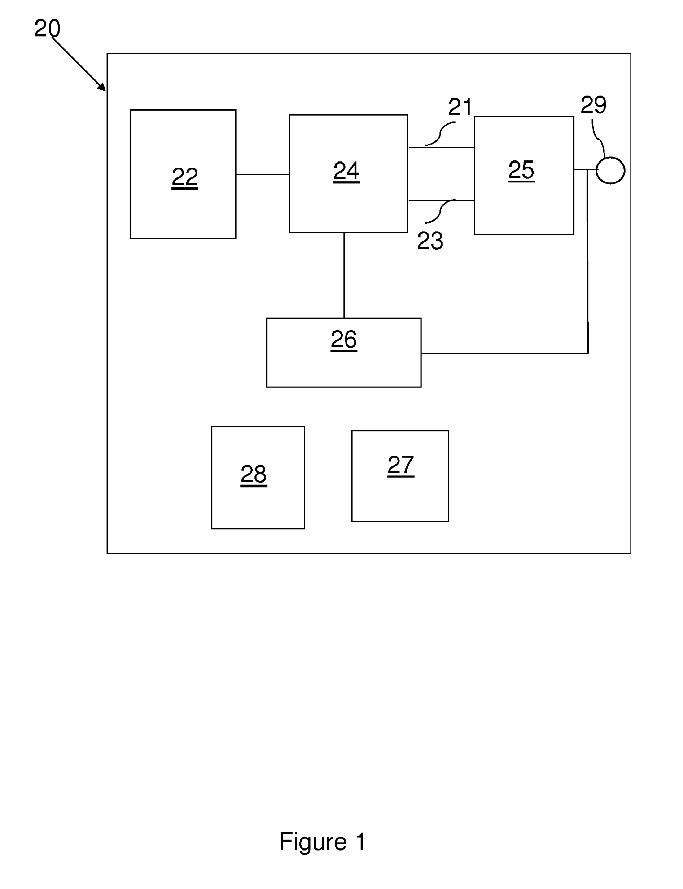

The potential operational modes of a detector are ordinarily implemented using variations in wiring *viz*. separate terminals for source and sink modes, or by using selection switches or selection links, which can be positioned to connect appropriate circuitry. In accordance with an aspect of the invention, apparatus is now described in reference to Figure 1, whereby the apparatus provides advantages, including automatic determination of the appropriate operational mode and switching to provide operation in the appropriate operational mode.

Figure 1 is a diagram of apparatus 20 according to an example of the invention.

In preferred embodiments the apparatus 20 is a gas detector. The gas detector 20 comprises a gas sensor 22 and its associated signal processing system. The gas sensor's 22 output is connected to switching means 24. The switching means 24 determines which of the signal output circuitry the processed signal is connected, i.e. the source or sink mode circuitry. Switching means 24 is connected to a voltage comparator 26. The switching means 24 is further connected to the current output circuitry 25, by inputs 21, 23, and has the means to either supply (source) current to the signal terminal 29 or draw (sink) current from the signal terminal 29, depending on which input 21, 23 the switching means 24 routes the processed sensor signal through. The apparatus 20 further comprises a memory 27 and a processor 28.

In further examples the gas sensor 22, switching means 24, current output circuitry 25 and voltage comparator 26 are configured to relay signals wirelessly between themselves, thereby having the advantage that the components can be arranged within the detector 20 in a way that suits the situation in hand. In yet further examples, the processor 28 and memory 27 are arranged to control the operation of the sensor 22, switching means 24, current output circuitry 25 and voltage comparator 28. The processor 28 and memory 27 are integrated into the apparatus 20. In further examples, the memory 27 and processor 28 are external to the apparatus 20.

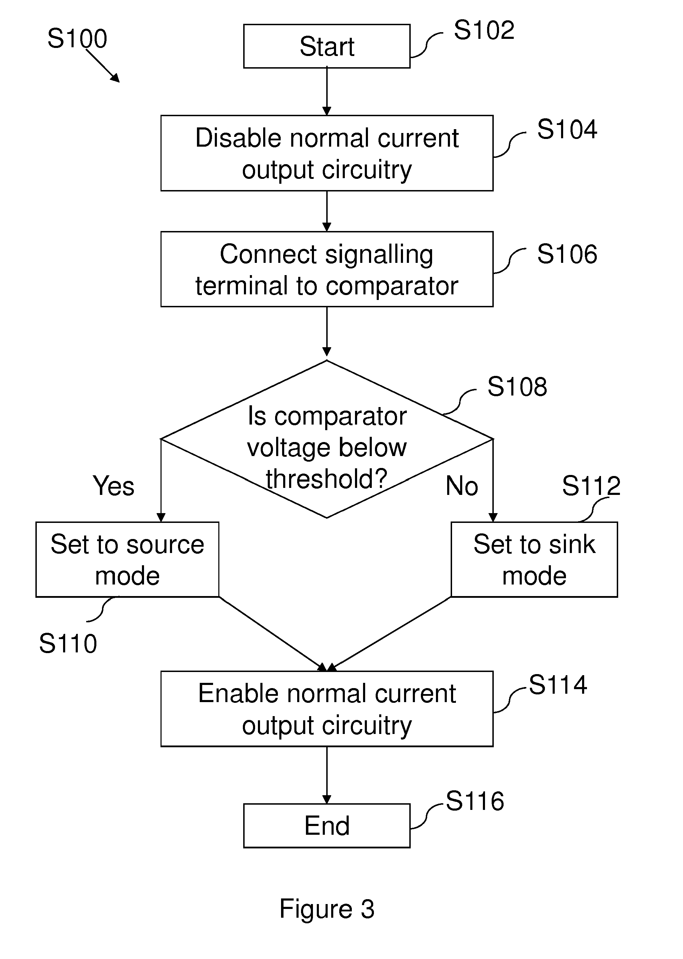

In operation, the apparatus/detector 20 is installed in an appropriate location, whereby the correct mode of operation of the detector 20 is determined. In use, the detector 20 compares the signal terminal's voltage to a threshold voltage to determine if the operational mode is a sink or source mode. The detector is further configured to automatically selectively switch to operate in one of sink mode or source mode, thereby enabling fast and effective switching of the mode of the detector 20 to the correct mode of operation with no user intervention. This process is described in detail with reference to Figure 3. An example of an appropriate location is an industrial plant, which may have many detectors, which in a preferred embodiment are gas detectors 20.

In further embodiments the gas detectors 20 relay information to a central location such as a computer that monitors the location in which the detectors are placed. In the same or further embodiments the gas detector is linked to an alarm system (not shown). In situations where detectors are relied on for safety reasons, it is important that they are operating correctly. Where many detectors need to be updated or replaced on a large scale, it is important to have a fast and reliable way to replace the detectors and to ensure that the operating mode is correct. The apparatus described in Figure 1 facilitates such an aim when it is operated in the manner described in relation to Figure 3.

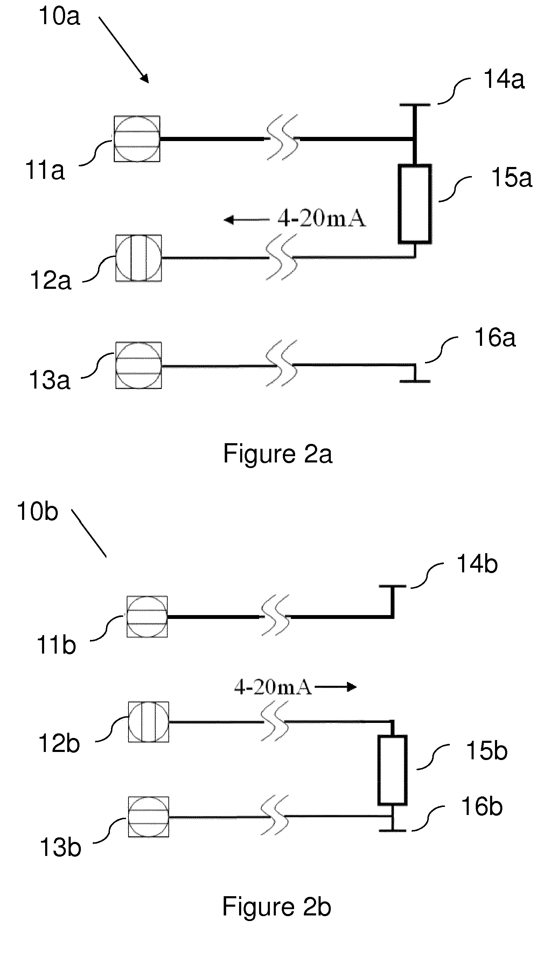

Figures 2a and 2b show a schematic representation of source mode and sink mode operation according to an example of the invention. Figure 2a shows source mode operation of a detector. There is shown a power terminal 11a, a signalling terminal 12a connected to a load 15a, which is in turn connected to a voltage supply 14a. There is also shown a ground supply 13a to the detector connected to ground 16a.

When a control panel (not shown) is configured to supply current (source) to the detector (not shown), provided the detector is not drawing any current, the voltage on the signalling terminal 12a will be virtually the same as the voltage supply 11a to the detector.

Figure 2b shows sink mode operation of a detector. There is shown a power terminal 11b, a signalling terminal 12b connected to a load 15b. The load 15b is further connected to a ground supply 16b. There is also shown a ground supply to the detector connected to ground 13b.

When a control panel (not shown) is configured to receive (sink) current supplied by the detector (not shown), provided the detector is not supplying any current, the voltage on the signalling terminal 12b will be virtually the same voltage as at the ground supply to the detector 13b.

The configuration described in relation to Figures 2a and 2b can be used to differentiate between sink mode and source mode states of operation. This is further described in relation to Figure 3, which is the implementation of a process for differentiating between source mode and sink mode and switching to operate in the appropriate mode.

Figure 3 is a flowchart S100 of a method according to an example of the invention. At step S102 the process starts. The process is initiated by the installation of apparatus 20 containing the gas sensor 22, the switching means 24, the current output circuitry 25 and the voltage comparator 26 into a system that has the capability to provide a voltage supply to the apparatus and that can receive an output signal from the apparatus.

At step S104, the normal current output circuitry 25 is disabled. By disabling the switching means 24, the correct operational state can be determined. The disabling of the switching means 24 stops any signal from the sensor 22 from reaching the current output circuitry 25. Therefore, as described with reference to Figures 2a and 2b, once the switching means is disabled the apparatus 20 is in a suitable state for signal terminal voltage to be analysed to determine the mode of operation(i.e. sink or source mode). Once the current output circuitry 25 has been disabled by disabling the switching means 24, the process moves to step S106.

At step S106, the signalling terminal 29 is connected to the comparator 26. This enables the comparator 26 to receive a voltage from the signalling terminal 29 that can be used to determine the operational requirements of the apparatus to which a detector 20 is to be connected.

At step S108 it is asked whether the voltage received at the comparator 26 is above or below a predetermined threshold. If the voltage at the comparator 26 is below a predetermined threshold, the process moves to step S110.

At step S110 the operational mode is set to the source mode using the switching means 24. If the voltage at the comparator 26, as measured at step S108, is not below the predetermined threshold, the process moves to step S112, whereby the operational mode is set to sink mode using the switching means 24. Once the operational mode has been set at either step S110 or S112, the process moves to step S114, whereby the normal current output circuitry is enabled. Once normal current output circuitry has been enabled, the signal from the gas sensor 22, which in an example is a conditioned sensor signal that has been processed at a processor 28, controls the current through the signal terminal 29 and the gas detector 20 operates normally. The process then ends at step S116, whereby the gas detector 20 has been configured to operate in accordance with the requirements of the system to which it has been connected.

In a preferred embodiment, the manner in which the appropriate mode of operation (sink or source mode) is determined is as described in reference to Figures 2a and 2b, whereby the voltage at the signalling terminal 12a that is connected to the 4 - 20 mA signal is compared with the voltage supply 11a to the gas detector 20. If it is similar to, or the same as, the voltage supply 11a to the gas detector 20, the mode of operation is source mode. If, however, the voltage at the signalling terminal 11b that is connected to the 4 - 20 mA signal is similar to, or the same as, the ground supply 13b to the gas detector 20, the mode of operation is sink mode.

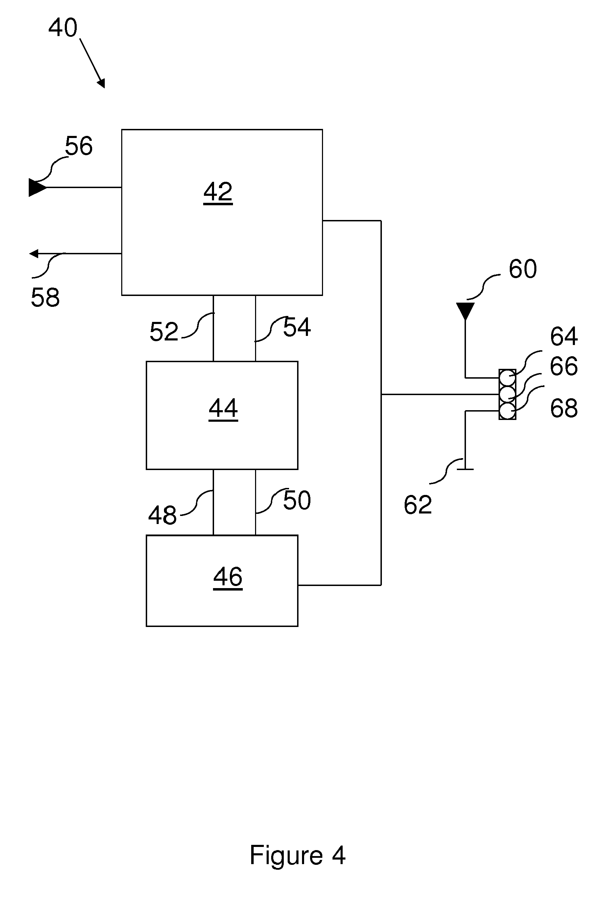

Such a comparison of voltage can be made by the comparator 26 at step S108 of the method detailed at flowchart S100. At step S108, the voltage received at the comparator 26, from the signalling terminal 29, is above or below a predetermined threshold. The predetermined threshold is set to determine whether the voltage supply to the gas detector 20 is similar to, or the same as, the voltage at the signalling terminal 29, or whether the voltage at the signalling terminal 29 is the same as, or similar to, the ground supply. Further details of how this step is performed are described in reference to Figure 4 below.

In situations where many gas detectors 20 are installed, for example in a large industrial plant, it is advantageous that the gas detectors 20 automatically are configured to operate in source or sink mode, depending on the requirements of the system. Further, when changes are made at a later stage, when the detectors 20 may have been operational for some time, there is no requirement to determine the configuration and rewire accordingly, because the gas detector 20 automatically determines and switches its own output circuitry to operate in either source mode or sink mode. This saves the user time and is more economically cost effective.

Preferably, the system to which the apparatus 20 is connected is a control panel. In further examples, numerous gas detectors 20 are connected to the control panel. In further examples, the control panel is any kind of conduit for receiving signals from the gas detectors 20. In further examples, the control panel supplies power to the gas detectors 20. In further examples, the power supply to the gas detectors 20 and the conduit for receiving signals from the detectors 20 are separate from one another.

In order to determine the appropriate mode of operation for a gas detector 20 being connected to a system and to further switch the mode of operation, suitable apparatus is required.

Figure 4 is a schematic of an apparatus for switching 40, according to an example of the invention.

Figure 4 shows a current output circuitry 42 that is electrically connected to mode selection logic 44. Mode selection logic 44 is electrically connected to a voltage comparator 46. The voltage comparator 46 is further connected to a signal terminal 66, preferably a 4 - 20 mA signal terminal 66 in a terminal block. Further in the terminal block are a ground terminal 68 connected to ground 62 and a voltage terminal 64 connected to a voltage 60.

A signal is sent from the voltage comparator 46 to the mode selection logic 44 to indicate whether the signal voltage is less than a reference line 48 or whether the signal voltage is greater than a reference line 50. This input to the mode selection logic 44 causes an appropriate signal to be sent from the mode selection logic 44 to the current output circuitry 42 at an appropriate time. The appropriate time is determined at the mode selection logic 44. The output enable 52 signal is sent once the voltage comparator 46 has provided the mode selection logic 44 with sufficient information to determine whether the mode of operation is sink mode or source mode.

Preferably the mode selection logic 44 comprises a timer and a latch. In further examples the mode selection logic 44 comprises software and a microcontroller, wherein the software and microcontroller are configured to provide timing and latch functions. In further examples, the microcontroller is configured to measure the voltage at the signalling terminal without the use of a comparator external to the microcontroller. Where the mode selection logic 44 comprises a timer and a latch, the timer is configured to begin a cycle when power is applied to the gas detector 20. The timer provides a signal to disable the normal current output circuitry. At the end of the timer cycle, a latch is enabled to record the state of the voltage comparator 26, 46 output. The output from the comparator 26, 46 is applied to the current output circuitry 25, 42 and the current output function is enabled.

In an example, the detector 20 forms part of a larger installation using many detectors 20. The detectors 20 form part of a system that is connected to a central computer. The central computer monitors the measurements from detectors 20. In a further example, the detectors 20 are connected to one or more alarms. The alarm system is controlled via a central computer. Advantageously, the detectors 20 are distributed over a large area and a central computer is used to monitor changes in the system and provide an alarm when the changes are measured.

## Claims

1. An apparatus for selectively switching a mode of operation of a detector, the apparatus comprising:
a detector configured for connection to a signalling terminal;
switching means for configuring the detector to operate in one of sink mode or source mode; and
a voltage comparator, wherein the apparatus is configured to selectively switch to operate in sink mode or source mode based on a comparison of a voltage from the signalling terminal received at the voltage comparator with a threshold voltage.

2. The apparatus according to claim 1, wherein the detector is a gas detector.

3. The apparatus according to claim 1, wherein the switching means comprises a timer and a latch.

4. The apparatus according to claim 1, wherein the switching means comprises a microcontroller configured to perform timing and latch functions.

5. The apparatus according to claim 1, wherein the signalling terminal is a 3 wire connection terminal.

6. The apparatus according to claim 1, wherein the signalling terminal is a 4 - 20 mA terminal.

7. The apparatus according to claim 1, wherein the switching means comprises mode selection logic.

8. The apparatus according to claim 1, wherein the apparatus is configured for connection to a central computer.

9. The apparatus according claim 1, wherein the apparatus is configured for connection to an alarm.

10. The apparatus according to claim 3, wherein the timer begins a cycle when power is applied to the detector and wherein at the end of the cycle the comparison of the voltage received at the voltage comparator with a threshold voltage is made.

11. A method for selectively switching a mode of operation of a detector, the method comprising:
receiving a voltage signal at a detector;
at a voltage comparator, making a comparison of the voltage signal received at the detector with a threshold voltage; and
based on the comparison, switching to operate the detector in one of sink mode or source mode.

12. The method according to claim 11, wherein the detector is a gas detector.

13. A system comprising a plurality of apparatuses as defined in claim 1.

14. The system of claim 13, wherein the plurality of apparatuses are configured for connection to a central computer.

15. The system of claim 13 wherein the plurality of apparatuses are configured for connection to an alarm.
